Europäisches Patentamt

European Patent Office

Office européen des brevets

(19)

(11) Veröffentlichungsnummer : **0 040 378**
**B1**

(12) **EUROPÄISCHE PATENTSCHRIFT**

(45) Veröffentlichungstag der Patentschrift :
02.05.84

(21) Anmeldenummer : 81103551.8

(22) Anmeldetag : 09.05.81

(51) Int. Cl.³ : **A 61 L 15/07, A 61 L 15/01**

(54) **Mischung für halbstarre medizinische Stützverbände, damit erhaltene medizinische Binde und Verfahren zu deren Herstellung.**

(30) Priorität : 17.05.80 DE 3018969

(43) Veröffentlichungstag der Anmeldung :
25.11.81 Patentblatt 81/47

(45) Bekanntmachung des Hinweises auf die Patenterteilung : 02.05.84 Patentblatt 84/18

(84) Benannte Vertragsstaaten :
AT BE DE FR GB IT LU NL SE

(56) Entgegenhaltungen :
CH-A- 282 212
DE-A- 1 767 434
DE-B- 1 282 228
FR-A- 1 227 448
GB-A- 498 975
US-A- 3 043 298

(73) Patentinhaber : Beiersdorf Aktiengesellschaft
Unnastrasse 48
D-2000 Hamburg 20 (DE)

(72) Erfinder : Pietsch, Hanns, Dr. Dipl.-Chem.
Mittelweg 25
D-2000 Hamburg 13 (DE)
Erfinder : Hohmann, Volker
Theodor Fontane Strasse 7a
D-2000 Norderstedt (DE)

EP 0 040 378 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Mischung für halbstarre medizinische Stützverbände, damit erhaltene medizinische Binde
und Verfahren zu deren Herstellung

Die Erfindung betrifft eine Zinkoxid enthaltende Mischung, welche sich insbesondere für die Herstellung von halbstarren Stützverbänden für medizinische Zwecke eignet, mit dieser Mischung erhältliche medizinische Binden und Herstellungsverfahren dafür.

Es sind bereits Zinkleimverbände bekannt, die aus Zinkoxid, Gelatine, Glycerin, Wasser und einem Konservierungsmittel bestehen. Neben der Gelatine können auch andere Naturstoffe wie Alginate, Gummi arabicum, und Agar-Agar als Bindemittel mitverwendet werden. Auch kann das Glycerin zum Teil durch andere mehrwertige Alkohole wie 1,2-Propylenglykol oder Sorbit ersetzt werden. Als Konservierungsmittel werden häufig Ester der p-Hydroxybenzoesäure oder Borsäure eingesetzt.

Diese « klassischen » Zinkleimverbände haben sich seit Jahrzehnten bei der Therapie von Beinleiden behaupten können. Sie werden, wie aus der Literatur bekannt ist, angewendet zur Behandlung von Thrombosen und abheilenden Beingeschwüren, als Stützverband bei kleineren Verletzungen und zur Verhinderung des Schwellens gebrochener Gliedmaßen nach Entfernen des Gipsverbandes, sowie als halbstarre Kompressionsverbände bei Varicosis, Ulcera, Phlebitis und Beinödemen, obwohl es eine Reihe von Ärzten gibt, die von der Verwendung von solchen Zinkleimbinden abraten.

Ein großer Nachteil dieser bekannten Zinkleimbinden besteht in ihren sehr langen Trockenzeiten nach dem Anlegen. Deswegen sind die Binden lange Zeit klebrig und schmierig.

Als besonders starkes Argument für die Verwendung des Zinkleimverbandes wird immer wieder seine Hautfreundlichkeit genannt, d. h. eine pflegende und heilende Wirkung auf die Haut. Es gibt aber eine Reihe von Patienten, und deren Zahl nimmt ständig zu, die die immer enthaltenen Konservierungsmittel nicht vertragen und starke Hautreizungen bekommen. Dadurch wird bei diesen Patienten die gerühmte Hautfreundlichkeit bzw. Hautverträglichkeit des Zinkleims aufgehoben oder sogar ins Gegenteil verkehrt !

Außerdem kommt es trotz des Vorhandenseins eines Konservierungsmittels, dessen Mengen gering zu halten man aus den oben genannten Gründen bemüht ist, immer wieder vor, daß z. B. bei längerer Lagerung oder bei ungenügender Verpackung die feuchte Binde durch Bakterien- oder Pilzbefall verdirbt, was sich durch unangenehmen Geruch und unschönes Aussehen sehr störend bemerkbar macht. Besonders unangenehm ist der Zersetzungsgeruch der Gelatine nach Ammoniak und Aminen, den man in unterschiedlicher Stärke, abhängig von Fabrikat und Lagerzeit, häufig bei Handelsprodukten feststellen kann.

Eine wirksame und gut zu handhabende Methode zur Abtötung von Keimen ist die Bestrahlung mit energiereichen Strahlen wie β- oder γ-Strahlen. Gegenüber der Gassterilisation weist sie den Vorteil auf, daß auch noch im verpackten Zustand eine Sterilisation vorgenommen werden kann. Dieser Vorteil ist besonders wertvoll, falls es sich bei der Verpackung um gasdichtes, also auch wasserdampfundurchlässiges, keimdichtes, also hermetisch abschließendes Material wie beispielsweise Glas oder Metall handelt. In diesem Fall ist eine nachträgliche Kontamination ausgeschlossen. Auch gegenüber der Dampfsterilisation weist die Strahlensterilisation Vorteile auf, weil das Material nicht durch hohe Temperaturen belastet wird.

Andererseits verändern sich eine Reihe von Materialien wie natürliche Bindemittel in störender Weise durch die Einwirkung von energiereichen Strahlen in der zur Sterilisation erforderlichen Dosis von etwa 2,5 Mrad. So gerinnt ein wäßriges Gelatine-Gel bei Bestrahlung oder Gummi arabicum beispielsweise entwickelt nach γ-Bestrahlung einen sehr unangenehmen, leicht stechenden Geruch. Bei anderen natürlichen Bindemitteln kommt es zu einem erheblichen Abfall der Viskosität.

Zinkleim, der Gelatine oder auch Beimischungen natürlicher Bindemittel enthält, hat den Nachteil, daß er nicht durch Bestrahlung sterilisiert werden kann, weil die dadurch hervorgerufene Koagulation des Gelatine-Gels begleitet von einer Abscheidung von Wasser, ein Modellieren bei der Anwendung der Masse oder Binde verhindert. Gerade diese Eigenschaft ist für das ordnungsgemäße Anlegen solcher Massen und Binden aber zwingend erforderlich, damit keine Druckstellen entstehen.

Die Herstellung herkömmlicher gelatinehaltiger Zinkleime erfordert, daß die Gelatine unter Erwärmen in Wasser gelöst werden muß, bevor die weiteren Komponenten eingearbeitet werden können. Auch dies ist im Hinblick auf eine rationelle Fertigung sehr nachteilig.

Aufgabe der Erfindung ist es, Zinkoxid enthaltende Mischungen und damit erhältliche Binden sowie einfache Verfahren zu deren Herstellung zu schaffen, die diese Nachteile nicht besitzen, sondern nach dem Anlegen schneller trocknen, bessere Hautverträglichkeit besitzen und sich einfacher konservieren oder sogar sterilisieren lassen, um sie auch lange Zeit lagerfähig zu machen. Sie sollen bei der Behandlung mit energiereicher Strahlung keine unvorteilhaften Veränderungen erfahren. Die Mischungen der Binden sollen eine möglichst cremige Konsistenz besitzen, damit bei dem Anlegen des Verbandes an Gliedmaße eine gute Modellierfähigkeit und damit gute Anpassung gegeben ist und ein glatter Verband erzielt wird.

Die Herstellung der Mischung und der Binde sollen bei niedriger Temperatur und auf herkömmliche Beschichtungsanlagen erfolgen und die Bestrahlung soll in üblichen Anlagen erfolgen können.

Die Aufgabe der Erfindung wird gelöst durch eine Zinkoxid enthaltende Mischung, insbesondere für die Herstellung von halbstarren Stützverbänden für medizinische Zwecke, die dadurch gekennzeichnet ist, daß sie aus

10-35 % Zinkoxid,
 2-15 % Celluloseäther,
10-35 % Glycerin,
30-75 % Wasser und
 0- 5 % Zusatzstoffen

besteht.

Vorzugsweise besteht diese Mischung aus

10-20 % Zinkoxid,
 3- 5 % Celluloseäther,
10-20 % Glycerin,
60-70 % Wasser und
 2- 4 % Zusatzstoffen.

Alle Angaben erfolgen in Gew.-%.

In einer besonders bevorzugten Ausführungsform der Erfindung werden diese Mischungen oder damit erhaltene Binden mit energiereicher Strahlung behandelt.

Das verwendete Zinkoxid wird als feinkörniges Pulver, vorzugsweise in der Pharmaqualität des Deutschen Arzneibuches eingesetzt, jedoch ist auch die Qualität « Weißsiegel » geeignet, die sich im Handel befindet.

Geeignete Celluloseäther sind Alkyläther, Arylalkyläther, Hydroxyalkyläther und Hydroxyalkyl-alkyläther der Cellulose. Bevorzugte Äther sind die Methylcellulose, Äthylcellulose, Benzylcellulose, Ethylmethylcellulose, Hydroxymethylcellulose, Hydroxyäthylcellulose, Hydroxypropylcellulose, Hydroxypropylmethylcellulose, Hydroxyäthylmethylcellulose sowie Äthylhydroxyäthylcellulose und Hydroxypropyläthylcellulose. Besonders bevorzugte Celluloseäther sind Methylhydroxyäthylcellulose, Methylhydroxypropylcellulose, Äthylhydroxyäthylcellulose und Hydroxypropylmethylcellulose. Alle Celluloseäther können entweder allein oder im Gemisch miteinander verwendet werden.

Derartige Celluloseäther gibt es in verschiedenen Typen, die sich in ihren mittleren Molgewichten unterscheiden und die eine unterschiedliche Viskosität in wäßriger Lösung aufweisen. Zur Charakterisierung wird üblicherweise die Viskosität einer zweiprozentigen wäßrigen Lösung herangezogen. Gut geeignet sind Celluloseäther, die in zweiprozentiger wäßriger Lösung eine Viskosität von 50-50 000 mPas ergeben. Vorzugsweise beträgt die Viskosität 50-12 000 mPas. Besonders bevorzugt werden Typen, die eine solche Viskosität von etwa 2 000 mPas aufweisen.

Bei Verwendung der bevorzugten Celluloseäther-Typen mit einer Viskosität von etwa 2 000 mPas beträgt der Celluloseätheranteil zweckmäßigerweise 3-5 %. Bei Typen mit höherer Viskosität genügen geringere Mengen, bei Typen mit niedrigerer Viskosität sind größere Mengen erforderlich.

Bei der Wahl der in der Viskosität unterschiedlichen Celluloseäther ist auch ein Viskositätsabfall zu berücksichtigen, der in unterschiedlichem Ausmaß, abhängig von der Bestrahlungsdosis, nach dem Bestrahlen der fertigen Mischung mit energiereicher Strahlung auftritt.

Vorzugsweise beträgt die Viskosität der erfindungsgemäßen Mischungen 1-100 Pas. Dies gilt auch für bestrahlte Massen.

Als Zusatzstoffe können weitere hydrophile makromolekulare Substanzen wie Polyvinylalkohol, Polyvinylpyrrolidon oder Polyhydroxyäthylmethacrylat, Polyäthylenoxid, Farbstoffe, Duftstoffe, Konservierungsmittel oder anorganische Verdickungsmittel wie Silikate oder Siliciumdioxid verwendet werden. Diese anorganischen feinteiligen Verdickungsmittel verleihen den Mischungen, bzw. den Binden sehr günstige thixotrope Eigenschaften.

In einer bevorzugten Ausführungsform der Erfindung enthält die erfindungsgemäße Mischung Silikate oder hochdisperses Siliciumdioxid. Ein Zusatz von 2 bis 4 % anorganischem Verdickungsmittel, vorzugsweise hochdispersem Siliciumdioxid mit großer Oberfläche (nach BET) von 100-400 $m^2/g$ und einem Schüttgewicht von etwa 60 g/l wird besonders bevorzugt. Sehr vorteilhaft ist die Verwendung von solchen anorganischen Verdickungsmitteln zur Kompensation des durch Bestrahlung hervorgerufenen Viskositätsabfalls. In dieser bevorzugten Ausführungsform der Erfindung ist es nicht unbedingt erforderlich, höherviskose Celluloseäther einzusetzen.

Weiterhin kann die Viskosität der Mischung durch Variation des Wassergehalts und des Zinkoxidanteils beeinflußt werden. Beispielsweise nimmt bei Verringerung der Wassermenge oder Erhöhung der Zinkoxidmenge die Viskosität zu.

Die Herstellung dieser erfindungsgemäßen Mischungen erfolgt durch Auflösen und Verrühren der Bestandteile bei Raumtemperatur. Zweckmäßigerweise gibt man Zinkoxid und den Celluloseäther in das Wasser-Glycerin-Gemisch. Zusatzstoffe wie die anorganischen Verdickungsmittel werden am besten zuerst in die Flüssigkeiten geben.

Mit diesen Mischungen sind die erfindungsgemäßen Binden erhältlich, die aus einem Träger bestehen, der einseitig oder beidseitig mit der Mischung beschichtet ist. Geeignete Träger sind textile Gewebe oder Vliese. Die Zinkoxid enthaltende Mischung dringt in die Zwischenräume der Trägermaterialien ein. Diese Binden sind zweckmäßigerweise zu Rollen aufgewickelt.

Als Träger kann das übliche Bindenmaterial verwendet werden wie : Mullbinden nach DIN 61631, entweder 20-fädig oder 24-fädig, mit Webkanten auf beiden Seiten, mit Kett- und Schußfäden, Baumwolle/Baumwolle, Zellwolle/Baumwolle oder Zellwolle/Zellwolle, gebleicht, ungebleicht oder gefärbt. Geeignet sind aber auch Mullbinden aus anderen Textilgarnen, z. B. aus Kunstfasern wie Polyester, Polyamid, aus Viskose oder aus Mischfasern. Weiterhin können auch elastische Gewebe, längselastische, querelastische sowie längs- und querelastische Gewebe verwendet werden.

Die Trägergewebe sollten vorzugsweise auf beiden Seiten Webkanten aufweisen, damit es beim Abwickeln der Binden nicht durch zerschnittene Kettfäden zur Bildung von Wulsten an den Kanten kommt, andernfalls sollten Kett- und Schußfäden durch eine geeignete Imprägnierung an den Kreuzungspunkten fixiert sein. Die Breite liegt zwischen 5 und 15 cm, vorzugsweise 7 und 10 cm. Um die Binden handlich abwickeln zu können, sollte der Kern einen nicht zu großen Außendurchmesser aufweisen ; er sollte zwischen 5 und 20 mm, vorzugsweise 8 und 15 mm betragen. Zum besseren Abrollen können die Kanten leicht abgerundete Wülste aufweisen. Das Material der Kerne kann aus Pappe oder Kunststoff bestehen, wobei die Pappe keinerlei ausblutende Farbstoffe enthalten sollte oder aber auch eine Lackierung oder eine andere Schutzschicht, z. B. durch eine Folie, gegen Ausbluten von Farbstoffen und Erweichung geschützt sein.

Um das Auffinden des Anfangs zu erleichtern, ist es vorteilhaft, eine Zunge bzw. Lasche vorzugsweise farbig aus Kunststoff oder imprägniertem Papier, beispielsweise PVC-Folie oder silikonisiertem Papier anzubringen, die 1-2 cm breit und 2-5 cm lang sein kann.

Um die erfindungsgemäße Mischung oder die damit hergestellte Binde lagerfähig zu halten, d. h. vor dem Austrocknen und vor Verkeimung zu schützen, sollte die Verpackung feuchtigkeitsdicht und keimdicht sein. Geeignete Verpackungsmaterialien sind verschweißbare Aluminiumfolien, wie polyäthylenbeschichtete oder polypropylenbeschichtete Aluminiumfolien, aber auch starre Behälter wie Dosen oder Schachteln aus Metall, Glas oder Verbundwerkstoffen, wobei die Deckel zusätzlich abgedichtet sein können, z. B. durch Versiegelung mit Klebestreifen.

Die Herstellung der Binden kann in bekannter Weise erfolgen, indem die erfindungsgemäße Mischung auf üblichen Streichanlagen einseitig oder beidseitig auf den Träger aufgebracht wird. In einer bevorzugten Ausführungsform werden Mullbinden in entsprechender Breite in einer Strichstärke von 120-200 g/m² gestrichen, aufgerollt und feuchtigkeitsdicht verpackt.

Überraschenderweise wird mit den erfindungsgemäßen Celluloseäthern ein wesentlich verbesserter « Zinkleim » erhalten. Die typische Eigenschaft der temperaturabhängigen Sol-Gel-Umwandlung der Gelatine, die mit einer starken Viskositätsänderung verbunden ist und auf die man glaubte nicht verzichten zu können, besitzen die Celluloseäther nicht. Ihre Verwendung in solchen Verbänden wurde daher bisher nicht in Betracht gezogen. Trotz dieses andersartigen Verhaltens lassen sich mit den erfindungsgemäßen Mischungen und Binden hervorragende halbstarre Verbände herstellen.

Ein wichtiger Vorteil der erfindungsgemäßen Massen und Binden ist die deutlich höhere Trockengeschwindigkeit. Etwa 1 bis 2 Stunden nach dem Anlegen sind die Verbände oberflächlich gut durchgetrocknet und damit mechanisch stabil und fühlen sich nicht mehr klebrig an.

Ein weiterer wesentlicher Vorteil ist die geringere Empfindlichkeit gegenüber Mikroorganismen, die sich in einer sehr viel besseren Haltbarkeit äußert. Ferner treten bei der Alterung keine unangenehmen Gerüche auf. Aufgrund der besseren Haltbarkeit kann bei kürzeren Aufbewahrungszeiten auf Konservierungsmittel ganz verzichtet werden. Lagerfähigere Produkte sind durch Zusätze von Konservierungsmitteln erhältlich, wobei die Mengen niedriger gewählt werden können als bei dem bekannten Zinkleim. Damit sind gerade die Hautverträglichkeit und Hautfreundlichkeit, d. h. die heilungsfördernde Wirkung, also sehr wichtige Eigenschaften, sehr verbessert worden.

Bei der Herstellung der Mischungen ist kein Erhitzen von Komponenten mehr erforderlich. Zur Verbesserung der Eigenschaften der Mischungen und der damit erhaltenen Binden ist eine Bestrahlung mit energiereichen Strahlen sehr günstig.

In einer besonders bevorzugten Ausführungsform der Erfindung wird daher auf den Zusatz von Konservierungsmitteln ganz verzichtet und die Mischung bzw. die damit hergestellte Binde einer Behandlung mit energiereicher Strahlung ausgesetzt. Hierzu eignen sich β- und γ-Strahlen. Bevorzugt wird die γ-Bestrahlung, wobei als Strahlenquelle $Co^{60}$ dient. Die Strahlungsdosis beträgt 0,5-5 Mrad, vorzugsweise 2,5 Mrad. Der durch die Bestrahlung hervorgerufene Viskositätsabfall kann durch die oben beschriebenen Maßnahmen, insbesondere durch Zusatz von anorganischen Verdickern, im gewünschten Maß kompensiert werden.

Die bestrahlten Massen, besonders die mit der bevorzugten Viskosität von 1-100 Pas, zeichnen sich durch eine sehr angenehme, cremige Konsistenz aus, die das Modellieren der angelegten Massen oder Binden weiter erleichtert. Die nicht bestrahlten Massen oder Binden sind gut schmiegsam und können ohnehin schon ohne Schwierugkeiten angelegt und angepaßt werden. Durch die Bestrahlung wird die Mischung bzw. Binde außerdem sterilisiert.

Herkömmliche Zinkleime lassen sich nicht in dieser Weise bestrahlen, da die verwendete Gelatine unter Wasserabscheidung koaguliert und der Zusammenhalt der ganzen Masse verloren geht, so daß diese Massen oder Binden nicht mehr verarbeitet werden können.

Vorzugsweise wird die Bestrahlung (γ-Strahlen) mit einer Dosis von etwa 2,5 Mrad durchgeführt, die zur Sterilisation von Masse oder Binde ausreicht. Solche konservierungsmittelfreien Produkte werden in einer bevorzugten Ausführungsform erhalten, indem man portionierte Gemische oder Mullbinden in zweckmäßigem Format, die in einer Strichstärke von 120-200 g/m² gestrichen und aufgerollt sind, sofort feuchtigkeitsdicht und keimdicht verpackt und sodann mit 0,5-5 Mrad, vorzugsweise 2,5 Mrad Strahlungsdosis sterilisiert. Zusätzlich werden mit diesem Schritt cremigere Massen erhalten. Zweckmäßigerweise wird die Bestrahlung spätestens nach 3 Tagen vorgenommen.

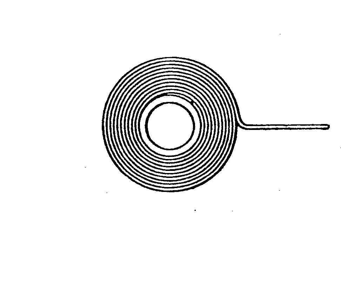

In der Zeichnung ist eine Ausführungsform der erfindungsgemäßen Binde im Querschnitt angegeben:

Ein Träger ist mit der erfindungsgemäßen Zinkoxid enthaltenden Mischung versehen und zu einer Rolle aufgewickelt.

Die folgenden Beispiele dienen zur Erläuterung der Erfindung.

Beispiel 1

Eine Zinkoxid enthaltende Mischung wird wie folgt hergestellt:

15 % (90 g) Zinkoxid (Pharmaqualität)
3,33 % (20 g) Methylhydroxyäthylcellulose (mit einer Viskosität von 2 000 mPas, in 2 %iger wäßriger Lösung bestimmt)
15 % (90 g) Glycerin (99 proz., DAB8)

werden nacheinander in 66,67 % (400 g) entionisiertes Wasser gerührt. Diese Mischung ist hervorragend geeignet zur Herstellung von halbstarren Stützverbänden.

Diese Zinkoxid enthaltende Mischung wird in einer üblichen Streichanlage, die zur Verarbeitung hochviskoser Massen eingerichtet ist, mit einer Strichstärke von 160 g/m² auf eine 10 cm breite Mullbinde, 20-fädig, mit Webkante auf beiden Seiten, gestrichen und auf einen Polystyrolkern von 10 mm Innendurchmesser und 14 mm Außendurchmesser aufgewickelt, in Wachspapier eingewickelt und sodann in polyäthylenbeschichtete Aluminiumfolie eingeschweißt.

Derartige Binden konnten nach mehrtägiger Lagerung bei Raumtemperatur gut verarbeitet werden und zeigten keinen störenden Geruch. In gleicher Weise verpackte und aufbewahrte Mischungen verhielten sich gleichartig.

Derartige Binden wurden folgendermaßen behandelt:

a) unmittelbar nach der Versiegelung wurde mit 2,5 Mrad (Co⁶⁰) bestrahlt.
b) keine Bestrahlung

| | Ergebnisse nach Lagerung | | | |
|---|---|---|---|---|
| | 2 Wochen | | 3 Monate | |
| Lagerung | 40 °C | 22 °C | 40 °C | 22 °C |
| a) bestrahlt | 1 | 1 | 1 | 1 |
| b) unbestrahlt | 2 | 2 | 2 | 2 |

Ergebnis 1 : Binde in Ordnung, kein Zersetzungsgeruch nach Amin, kein sichtbarer Pilzbefall
Ergebnis 2 : Binde enthält kreisförmige Pilzhöfe,
   Geruch : bei geringem Befall keiner, bei stärkerem
   Befall : dumpf, « kellerartig »
Die bestrahlten Binden besitzen eine angenehm cremige Konsistenz.

Beispiel 2-5

Es wurden folgende Zinkoxid enthaltenden Zubereitungen wie in Beispiel 1 angegeben hergestellt und zu Binden wie vorstehend beschrieben verarbeitet :

13,33 % (240 g) Zinkoxid (pharm.)
5 % (90 g) Celluloseäther
15 % (270 g) Glycerin (99 %)
66,67 % (1 200 g) entionisiertes Wasser

Als Cellulosederivate wurden verwendet :

| Beispiel | Celluloseäther | Viskosität der 2 %igen Lösung |
|---|---|---|
| 2 | Äthylhydroxyäthylcellulose | 50 mPas |
| 3 | Äthylhydroxyäthylcellulose | 300 mPas |
| 4 | Äthylhydroxyäthylcellulose | 700 mPas |
| 5 | Hydroxypropylmethylcellulose | 50 mPas |

Die entsprechenden Binden wurden sowohl bestrahlt (2,5 Mrad, γ-Strahlung) als auch unbestrahlt gelagert. Bedingungen und Ergebnisse waren wie in Beispiel 1 : sämtliche bestrahlten Muster waren nach 2 Wochen (40 °C, 22 °C) in Ordnung, während die unbestrahlten Schimmelpilz-Kolonien zeigten.

Zur Ermittlung der weiteren Eigenschaften wurden die Binden um ein Modellbein gewickelt. Nach 90 Minuten hinterließen die Bindenoberflächen keine Rückstände beim Anfassen und wurden als oberflächlich trocken beurteilt.

## Beispiel 6-12

Es wurden folgende Zinkoxid enthaltenden Zubereitungen wie in Beispiel 1 beschrieben hergestellt, wobei aber am Anfang $SiO_2$ zugegeben wurde, und zu Binden wie vorstehend verarbeitet :

14   % (268,2 g) Zinkoxid (pharm.)
3,33 % (60 g) Celluloseäther
15   % (270 g) Glycerin (99 %ig)
66,67 % (1 200 g) entionisiertes Wasser
1    % (18 g) hochdisperses Siliciumdioxid (Schüttgewicht 60 g/l, BET-Oberfläche 200 $m^2/g$)

Als Cellulosederivate wurden verwendet :

| Beispiel | Celluloseäther | Viskosität der 2 %igen Lösung |
|---|---|---|
| 6 | Methylhydroxypropylcellulose | 1 000 mPas |
| 7 | Methylhydroxypropylcellulose | 2 000 mPas |
| 8 | Methylhydroxypropylcellulose | 10 000 mPas |
| 9 | Methylhydroxypropylcellulose | 30 000 mPas |
| 10 | Äthylhydroxyäthylcellulose | 2 000 mPas |
| 11 | Äthylhydroxyäthylcellulose | 7 000 mPas |
| 12 | Äthylhydroxyäthylcellulose | 12 000 mPas |

Die entsprechenden Binden wurden sowohl bestrahlt (2,5 Mrad, γ-Strahlung) als auch unbestrahlt gelagert. Bedingungen und Ergebnisse waren wie in Beispiel 1 beschrieben : sämtliche bestrahlten Muster waren nach 2 Wochen (40 °C, 22 °C) in Ordnung, während die unbestrahlten Schimmelpilz-Kolonien zeigten.

Zur Ermittlung der Verarbeitungs- und Trocknungseigenschaften wurden die Binden um ein Modellbein gewickelt. Sie zeigten dabei ein hervorragendes cremiges, glattes Modellierverhalten. 60-90 Minuten nach Anlegen der Verbände blieb nach Berühren kein Rückstand auf der Hand, so daß die Binden als oberflächlich trocken angesehen wurden.

## Beispiel 13-16

Es wurden Zubereitungen wie in Beispiel 2 beschrieben, bestehend aus 3,33 % Methylhydroxypropylcellulose (Viskosität einer 2 % Lösung 2 000 mPas), 15 % Glycerin, 66,67 % Wasser sowie folgenden Bestandteilen, hergestellt :

(Siehe Tabelle Seite 7 f.)

| Beisp. | Zinkoxid in % | Hochdisperses $SiO_2$, in % | Polyäthylen-oxid (1), in % | Viskosität [Pas] (2) | |
|---|---|---|---|---|---|
| | | | | vor 2,5 Mrad $\gamma$-Strahlg. | nach 2,5 Mrad $\gamma$-Strahlg. |
| 13 | 14,83 | — | 0,17 | 15 | 0,18 |
| 14 | 11,83 | 3 (3) | 0,17 | 52 | 28 |
| 15 | 12,00 | 3 (3) | — | 55 | 26 |
| 16 | 11,00 | 4 (4) | — | 32 | 7 |

(1) Mittleres Molgewicht ca. $2 \cdot 10^6$ Dalton.
(2) Gemessen mit dem « Viskotester VT-02 » der Fa. Haake.
(3) Schüttgewicht 60 g/l, BET-Oberfläche 300 m²/g.
(4) Schüttgewicht 60 g/l, BET-Oberfläche 200 m²/g.

Diese Massen wurden zu Binden wie in den vorherigen Beispielen verarbeitet, verpackt, mit 2,5 Mrad $\gamma$-Strahlung bestrahlt und gelagert. Die Binden waren nach 3 Monaten bei Raumtemperatur und 40 °C in Ordnung.

Mit Binden entsprechend Beispiel 15 wurde eine 37 jährige Patientin mit Venenentzündung und Thrombose während eines halben Jahres behandelt. Die Binden ließen sich hervorragend anlegen, waren gut verstreichbar, von angenehmen Geruch und waren je nach relativer Luftfeuchtigkeit nach 60 bis 90 Minuten oberflächlich trocken.

Die Binden erfüllten voll ihren therapeutischen Zweck. Es kam zu einem Rückgang der Venenentzündung. Während der Behandlung wurde keine Hautreizung beobachtet. Das Verhalten der Binde wurde vom behandelnden Arzt hervorragend beurteilt.

## Beispiel 17

Eine Zinkoxid enthaltende Mischung wird wie folgt hergestellt :

15 % Zinkoxid (pharm.)
25 % Glycerin
11 % Äthylhydroxyäthylcellulose (mit einer Viskosität von 50 mPas, in 2 %iger Lösung bestimmt)

werden nacheinander in 49 % Wasser gerührt.

Die mit dieser Mischung wie in Beispiel 1 beschrieben hergestellten Binden ergaben gut geeignete halbstarre Stützverbände. Zusätzlich mit 2,5 Mrad $\gamma$-Strahlung bestrahlten Binden zeichneten sich durch eine cremige Konsistenz aus und ließen sich gut verarbeiten.

## Beispiel 18

Eine Zinkoxid enthältende Mischung wird wie folgt hergestellt :

20 % Zinkoxid (pharm.)
10 % Methylhydroxyäthylcellulose (mit einer Viskosität von 40 000 mPas in 2 %iger Lösung)

werden in ein Gemisch von 20 % Glycerin und 50 % Wasser gerührt. Mit dieser Mischung wurden, wie in Beispiel 1 angegeben, Binden hergestellt und mit 3,5 Mrad $\gamma$-Strahlung bestrahlt.

## Vergleichsbeispiel

Zum Vergleich wurde eine Zinkleimbinde nach konventioneller Rezeptur hergestellt :

## Bestandteile

2,33 % (14 g) Gelatine « Super Platin »
15,67 % (94 g) Glycerin (99 %)
15,33 % (92 g) Zinkoxid pharm.
66,67 % (400 g) Aqua dest.

In das auf 70 °C erwärmte Wasser wurde vorsichtig die Gelatine eingerührt und dann die Lösung unter Rühren abgekühlt. Sodann wurden nacheinander die übrigen Bestandteile hineingerührt. Aus diesem Zinkleim wurde eine Binde gestrichen wie in den vorstehenden Beispielen.

Beim Bestrahlen der verpackten Binden mit 2,5 Mrad $\gamma$-Strahlen kam es zur Koagulation des Zinkleims, sie war « käsig » und das Wasser ließ sich herausdrücken. Diese Binde konnte nicht verarbeitet werden.

Das gleiche wurde an einer handelsüblichen Zinkleimbinde beobachtet.

**Ansprüche**

1. Zinkoxid enthaltende Mischung, insbesondere für die Herstellung von halbstarren Stützverbänden für medizinische Zwecke, dadurch gekennzeichnet, daß sie aus

10-35 % Zinkoxid,
2-15 % Celluloseäther,
10-35 % Glycerin,
30-75 % Wasser und
0- 5 % Zusatzstoffen

besteht.

2. Mit einer Zinkoxid enthaltenden Mischung beschichtete Binde für die Herstellung von halbstarren Stützverbänden für medizinische Zwecke, dadurch gekennzeichnet, daß sie aus einem Träger besteht, der einseitig oder beidseitig mit einer Mischung aus

10-35 % Zinkoxid
2-15 % Celluloseäther,
10-35 % Glycerin,
30-75 % Wasser und
0- 5 % Zusatzstoffen

versehen ist.

3. Zinkoxid enthaltende Mischung und damit erhaltene Binde, insbesondere für die Herstellung von halbstarren Stützverbänden für medizinische Zwecke, dadurch gekennzeichnet, daß die Mischung aus

10-35 % Zinkoxid,
2-15 % Celluloseäther,
10-35 % Glycerin,
30-75 % Wasser und
0- 5 % Zusatzstoffen

besteht und daß sie, bzw. die Binde, mit energiereicher Strahlung bestrahlt ist.

4. Mischung oder Binde gemäß Anspruch 3, dadurch gekennzeichnet, daß sie mit γ-Strahlung bestrahlt ist.

5. Mischung und Binde gemäß den Ansprüchen 1 bis 4, dadurch gekennzeichnet, daß der Celluloseäther in 2 %iger wäßriger Lösung eine Viskosität von 50-50 000 mPas besitzt.

6. Mischung und Binde gemäß den Ansprüchen 1 bis 5, dadurch gekennzeichnet, daß die Celluloseäther Methylhydroxyäthylcellulose, Äthylhydroxyäthylcellulose, Hydroxypropylmethylcellulose und/oder Methylhydroxypropylcellulose sind.

7. Mischung und Binde gemäß den Ansprüchen 1 bis 6, dadurch gekennzeichnet, daß die Mischung eine Viskosität von 1-100 Pas besitzt.

8. Mischung und Binde gemäß Ansprüchen 1 bis 7, dadurch gekennzeichnet, daß die Mischung ein anorganisches Verdickungsmittel enthält.

9. Mischung und Binde gemäß Anspruch 8, dadurch gekennzeichnet, daß das Verdickungsmittel hochdisperses Siliciumdioxid ist.

10. Binde gemäß Ansprüchen 2-9, dadurch gekennzeichnet, daß der Träger Mull ist.

11. Binde gemäß den Ansprüchen 2-9, dadurch gekennzeichnet, daß der Träger ein elastisches Gewebe oder Vlies ist.

12. Binde gemäß den Ansprüchen 2-11, dadurch gekennzeichnet, daß die Auftragsstärke der Mischung 120-200 g/m², vorzugsweise 160 g/m², beträgt.

13. Mischung oder Binde gemäß Anspruch 3, dadurch gekennzeichnet, daß sie mit einer Dosis von 0,5-5 Mrad, vorzugsweise 2,5 Mrad, bestrahlt sind.

14. Verfahren zur Herstellung von mit einer Zinkoxid enthaltenden Mischung beschichteten Binde für halbstarre medizinische Stützverbände, dadurch gekennzeichnet, daß man eine Mischung aus

10-35 % Zinkoxid,
2-15 % Celluloseäther,
10-35 % Glycerin,
30-75 % Wasser und
0- 5 % Zusatzstoffen

## 0 040 378

einseitig oder beidseitig auf einen Träger aufbringt.

15. Verfahren zur Herstellung einer Binde gemäß Anspruch 14, dadurch gekennzeichnet, daß man die Mischung einseitig oder beidseitig auf einen Träger aufträgt und mit energiereicher Strahlung bestrahlt.

16. Verfahren zur Herstellung einer Binde gemäß Anspruch 15, dadurch gekennzeichnet, daß man die Mischung aufträgt und mit γ-Strahlen bestrahlt.

**Claims**

1. Mixture containing zinc oxide, in particular for producing semi-rigid fixed dressings for medical purposes, characterised in that it consists of

10-35 % of zinc oxide,
2-15 % of cellulose ethers,
10-35 % of glycerol,
30-75 % of water and
0- 5 % of additives.

2. Bandage, coated with a mixture containing zinc oxide, for producing semi-rigid fixed dressings for medical purposes, characterised in that it consists of a carrier which is provided on one side or both sides with a mixture of

10-35 % of zinc oxide,
2-15 % of cellulose ethers,
10-35 % of glycerol,
30-75 % of water and
0- 5 % of additives.

3. Mixture containing zinc oxide and bandage obtained with this mixture, in particular for producing semi-rigid fixed dressings for medical purposes, characterised in that the mixture consists of

10-35 % of zinc oxide,
2-15 % of cellulose ethers,
10-35 % of glycerol,
30-75 % of water and
0- 5 % of additives

and that it, or the bandage, has been irradiated with high-energy radiation.

4. Mixture or bandage according to Claim 3, characterised in that it has been irradiated with γ-radiation.

5. Mixture and bandage according to Claims 1 to 4, characterised in that the cellulose ether in a 2 % aqueous solution has a viscosity of 50-50,000 mPas.

6. Mixture and bandage according to Claims 1 to 5, characterised in that the cellulose ethers are methylhydroxyethylcellulose, ethylhydroxyethylcellulose, hydroxypropylmethylcellulose and/or methylhydroxypropylcellulose.

7. Mixture and bandage according to Claims 1 to 6, characterised in that the mixture has a viscosity of 1-100 Pas.

8. Mixture and bandage according to Claims 1 to 7, characterised in that the mixture contains an inorganic thickener.

9. Mixture and bandage according to Claim 8, characterised in that the thickener is highly disperse silica.

10. Bandage according to Claims 2-9, characterised in that the carrier is gauze.

11. Bandage according to Claims 2-9, characterised in that the carrier is an elastic fabric or nonwoven.

12. Bandage according to Claims 2-11, characterised in that the amount of mixture applied is 120-200 g/m², preferably 160 g/m².

13. Mixture or bandage according to Claim 3, characterised in that they have been irradiated with a dose of 0.5-5 Mrad, preferably 2.5 Mrad.

14. Method for producing a bandage, coated with a mixture containing zinc oxide, for semi-rigid medical fixed dressings, characterised in that a mixture of

10-35 % of zinc oxide,
2-15 % of cellulose ethers,
10-35 % of glycerol,
30-75 % of water and
0- 5 % of additives,

9

is applied to one side or both sides of a carrier.

15. Method for producing a bandage, according to Claim 14, characterised in that the mixture is applied to one side or both sides of a carrier and the carrier is then irradiate with high-energy radiation.

16. Method for producing a bandage, according to Claim 15, characterised in that the mixture is applied and the bandage is then irradiated with $\gamma$-radiation.


**Revendications**

1. Mélange contenant de l'oxyde de zinc, en particulier, pour la fabrication de pansements supports semi-rigides à usage médical, caractérisé en ce qu'il comprend

10-35 % d'oxyde de zinc,
2-15 % d'éther de cellulose,
10-35 % de glycérine,
30-75 % d'eau et
0- 5 % d'additifs.

2. Bandage enduit d'un mélange contenant de l'oxyde de zinc pour la fabrication de pansements supports semi-rigides à usage médical, caractérisé en ce qu'il est constitué d'un support sur une ou les deux faces duquel est appliqué un mélange comprenant

10-35 % d'oxyde de zinc,
2-15 % d'éther de cellulose,
10-35 % de glycérine,
30-75 % d'eau et
0- 5 % d'additifs.

3. Mélange contenant de l'oxyde de zinc et bandage obtenu avec ce mélange, en particulier, pour la fabrication de pansements supports semi-rigides à usage médical, caractérisés en ce que le mélange comprend

10-35 % d'oxyde de zinc,
2-15 % d'éther de cellulose,
10-35 % de glycérine,
30-75 % d'eau et
0- 5 % d'additifs,

tandis que ce mélange ou le bandage est irradié par un rayonnement riche en énergie.

4. Mélange ou bandage suivant la revendication 3, caractérisé en ce qu'il est irradié par des rayons gamma.

5. Mélange et bandage suivant les revendications 1 à 4, caractérisés en ce que l'éther de cellulose a, en solution aqueuse à 2 %, une viscosité de 50-50 000 mPa.s.

6. Mélange et bandage suivant les revendications 1 à 5, caractérisés en ce que les éthers de cellulose sont la méthylhydroxyéthyl-cellulose, l'éthylhydroxyéthyl-cellulose, l'hydroxypropylméthylcellulose et/ou la méthylhydroxypropyl-cellulose.

7. Mélange et bandage suivant les revendications 1 à 6, caractérisés en ce que le mélange a une viscosité de 1-100 Pa.s.

8. Mélange et bandage suivant les revendications 1 à 7, caractérisés en ce que le mélange contient un agent épaississant inorganique.

9. Mélange et bandage suivant la revendication 8, caractérisés en ce que l'agent épaississant est le dioxyde de silicium fortement dispersé.

10. Bandage suivant les revendications 2-9, caractérisé en ce que le support est la mousseline

11. Bandage suivant les revendications 2-9, caractérisé en ce que le support est un voile ou un tissu élastique.

12. Bandage suivant les revendications 2-11, caractérisé en ce que le mélange est appliqué à raison de 120-200 g/m$^2$, de préférence, à raison de 160 g/m$^2$.

13. Mélange ou bandage suivant la revendication 3, caractérisé en ce qu'on l'irradie avec une dose de 0,5-5 Mrad, de préférence, de 2,5 Mrad.

14. Procédé de fabrication d'un bandage enduit d'un mélange contenant de l'oxyde de zinc pour pansements supports semi-rigides à usage médical, caractérisé en ce que, sur une ou les deux faces d'un support, on applique un mélange comprenant :

10-35 % d'oxyde de zinc,
2-15 % d'éther de cellulose,

10-35 % de glycérine,
30-75 % d'eau et
0- 5 % d'additifs.

15. Procédé de fabrication d'un bandage suivant la revendication 14, caractérisé en ce qu'on applique le mélange sur une ou les deux faces d'un support et on l'irradie avec un rayonnement riche en énergie.

16. Procédé de fabrication d'un bandage suivant la revendication 15, caractérisé en ce qu'on applique le mélange et on l'irradie avec des rayons gamma.